# EUROPÄISCHE PATENTANMELDUNG

(11) **EP 0 607 749 A1**
(43) Veröffentlichungstag der Anmeldung: **27.07.1994**
(21) Anmeldenummer: 93810040.1
(22) Anmeldetag: 21.01.1993
(51) Int. Cl.: A61F 2/42

(54) **Künstliches Handgelenk**

(71) Anmelder: SULZER Medizinaltechnik AG, CH-8404 Winterthur (CH); ALLO PRO AG, CH-6340 Baar (CH)
(72) Erfinder: Gontran, Sennwald, Dr. med., CH-9000 St. Gallen (CH)
(74) Vertreter: Hammer, Bruno, Dr.

(57) **Zusammenfassung**

Der künstliche Gelenkanteil besteht aus einem distalen Tragkopf (6), welcher eine ballige Aussenfläche aufweist und mit Befestigungsmitteln (5) am Capitate (3) verankert ist. Eine als Gegenfläche (7) vorgesehene Führungsfläche, welche am Radius (1) verankert ist, weist die Form einer Rinne (16) auf, welche neben beliebigen Schwenkbewegungen des Tragkopfes (6) dessen Verschiebung quer zur Längsachse (15) des Radius (1) in einer von Radius (1) und Ulna (2) gebildeten Ebene ermöglicht. Da nur ein geringer Anteil der Handgelenkknochen entfernt wird, bleibt der Bandapparat erhalten und ermöglicht eine offene Führung.

## Beschreibung

Die Erfindung handelt von einem künstlichen Handgelenk zur Erzeugung einer Flexions- und einer Extensionsbewegung mit einem Tragkopf und mit einer Gegenfläche, die proximal am Radius verankert ist, wobei Handgelenkknochen entfernt worden sind.

Ein künstliches Handgelenk ist in der Patentschrift US 4,040,130 in Form eines beschränkten Kugelgelenks gezeigt. Eine Gelenkkugel ist dort über ein Joch und Knochennägel mit mehreren Metakarpal Knochen verbunden. Die Bewegung der Gelenkkugel ist gegenüber einer Zwischenschale, und die der Zwischenschale gegenüber der im Radius verankerten Lagerschale durch kreuzförmig zueinander angeordneten Nuten, in denen Führungsknochen nicht verdrehbar gleiten, beschränkt. Das Gelenk erlaubt die Schwenkung um zwei starre Achsen, die sich im Kugelgelenk kreuzen. Das Einsetzen eines derartigen Implantats stellt für ein Handgelenk einen grossen Eingriff dar, der nicht bei allen Erkrankungen des Handgelenks gerechtfertigt ist. Gerade bei arthritischen Erkrankungen des Handgelenks, bei denen eine Verringerung der Schmerzen gegenüber einer beschränkten Funktion des Handgelenks im Vordergrund steht, wäre ein reduzierter Eingriff von Vorteil. Die Erfindung hat daher die Aufgabe, mit wenig Verlust an Knochenmaterial ein Implantat zu schaffen, bei dem die wesentlichen Bewegungen des Handgelenks erhalten bleiben. Diese Aufgabe wird mit den Kennzeichen des unabhängigen Anspruchs 1 gelöst.

Die Erfindung hat den Vorteil, dass es sich um einen kleineren Eingriff handelt, indem hauptsächlich in der proximalen Karpalreihe Scarphoid, Lunatum und Triquetrum entfernt werden, während der Bandapparat weitgehend erhalten bleibt und nach einer natürlichen Verkürzung alle wesentlichen Funktionen weiterhin erfüllen kann. Ausserdem entsteht eine geringere Versteifung des Handgelenks, indem die natürlichen Bewegungsmöglichkeiten zwischen den verbleibenden Handgelenkknochen und den Metakarpals erhalten bleiben, während die Führung des implantierten Gelenkanteils analog zum natürlichen Gelenk von der Streckung von Sehnen und Bändern abhängig ist.

Der künstliche Gelenkanteil besteht aus einem distalen Tragkopf, welcher eine ballige Aussenfläche aufweist und mit Befestigungsmitteln am Capitate verankert ist. Eine als Gegenfläche vorgesehene Führungsfläche, welche am Radius verankert ist, weist die Form einer Rinne auf, welche neben beliebigen Schwenkbewegungen des Tragkopfes dessen Verschiebung quer zur Längsachse des Radius in einer von Radius und Ulna gebildeten Ebene ermöglicht. Da nur ein geringer Anteil der Handgelenkknochen entfernt wird, bleibt der Bandapparat erhalten und ermöglicht eine offene Führung.

Weitere vorteilhafte Weiterbildungen sind mit den Kennzeichen der Unteransprüche 2 bis 4 aufgeführt.

Im folgenden wird die Erfindung anhand von einem Ausführungsbeispiel erklärt. Es zeigt:

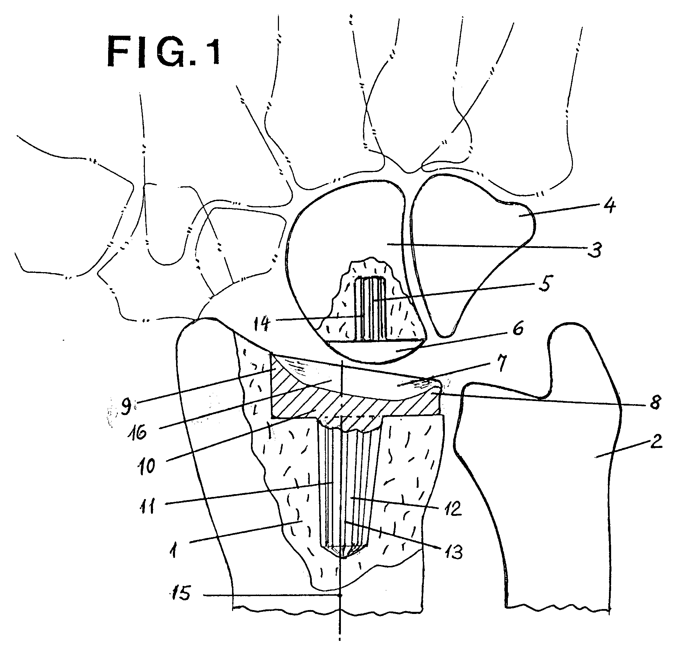

- Fig. 1: schematisch eine Draufsicht auf den vergrösserten Ausschnitt eines Handgelenks in der von Radius und Ulna gebildeten Ebene.

Die Figur 1 zeigt ein Handgelenk bei dem in der proximalen Karpalreihe Scaphoid, Lunatum und Triquetrum entfernt worden sind. Am Radius 1 mit Längsachse 15 ist eine Plattform 10 auf einer Resektionsfläche mit einem Verankerungsmittel 11 befestigt, das als Zapfen 12 mit Längsrippen 13 ausgeführt ist. Die Plattform 10 weist gegen distal zu einem Tragkopf 6 eine Gegenfläche 7 auf, die in Form einer Rinne 16 quer zur Längsachse 15 des Radius 1 und Ulna 2 gebildeten Ebene verläuft. Der Tragkopf 6 ist ballig und vorzugsweise als Teil einer Kugelfläche ausgeführt, die in jeder Position längs der Rinne 16 in den Auflagepunkten beliebig geschwenkt werden kann. Der Tragkopf 6 liegt mit einer Auflagefläche an einer passenden Resektionfläche des Capitate 3 auf und ist mit einem zylindrischen Stift mit Längsrippen 14 als Befestigungsmittel 5 im Capitate 3 verankert. Die Figur 1 zeigt eine gestreckte Position unmittelbar nach der Implantation, bei der der Tragkopf 6 von der Rinne 16 abgehoben ist, weil sich Bänder und Sehnen noch nicht verkürzt haben. Unter dem späteren Zug des Bandapparates liegt der Tragkopf 6 in der Rinne 16 auf und kann je nach Zug geschwenkt und/oder längs der Rinne 16 verschoben werden. Die Längsverschiebung ist jeweils durch eine hochgezogene Kante 8, 9 am Ende der Rinne 16 begrenzt. Soweit es der Bandapparat erlaubt, ist auch eine begrenzte Schwenkung des Tragkopfes 6 relativ zur Gegenfläche 7 um eine zur Längsachse 15 parallele Rotationsachse möglich. Dadurch, dass Hamate 4 und die mit durchbrochenen Linien angedeuteten Metakarpals unverändert bleiben, behält das Handgelenk seine Beweglichkeit. Da keine zwangsläufige Verbindung zwischen Tragkopf 6 und Plattform 10 besteht, kann jedes der beiden Teile in abgewinkelter Gelenkstellung unabhängig vom anderen in seinem Knochenbett befestigt werden. Die geringen Abmessungen der beiden Teile gestatten es, dabei schonend mit dem Bandapparat umzugehen.

## Patentansprüche

1. Künstliches Handgelenk zur Erzeugung einer Flexions- und einer Extensionsbewegung mit einem distalen Tragkopf (6) und mit einer Gegenfläche (7), die proximal am Radius (1) verankert ist, wobei Handgelenkknochen entfernt worden sind, dadurch gekennzeichnet, dass der Tragkopf (6) mit Befestigungsmitteln (5) am Capitate (3) verankert ist und mit einer balligen Aussenfläche in einer Gegenfläche (7) in Form einer Rinne (16) quer zur Längsachse (15) des Radius (1) in einer von Radius (1) und Ulna (2) gebildeten Ebene verschiebbar ist.

2. Künstliches Handgelenk nach Anspruch 1, dadurch gekennzeichnet, dass Anfang und Ende der Rinne (16) durch hochgezogene Kanten (8, 9) begrenzt sind.

3. Künstliches Handgelenk nach Anspruch 1 oder 2, dadurch gekennzeichnet, dass die Rinne (16) aus einer Plattform (10) herausgearbeitet ist, welche als Verankerungsmittel (11) einen Zapfen (12) mit Längsrippen (13) aufweist.

4. Künstliches Handgelenk nach einem der Ansprüche 1 bis 3, dadurch gekennzeichnet, dass der Tragkopf (6) relativ zur Gegenfläche (7) um eine zur Längsachse (15) parallele Rotationsachse begrenzt schwenkbar ist.
